# FASCICULE DE BREVET EUROPEEN

(11) **EP 0 514 263 B1**
(45) Date de publication et mention de la délivrance du brevet: **28.08.1996**
(21) Numéro de dépôt: 92401304.8
(22) Date de dépôt: 13.05.1992
(51) Int. Cl.: A61F 2/16

(54) **Dispositif intra-oculaire implantable dans le sac capsulaire**
Intraokulare Vorrichtung für die Implantation in dem Kapelsack
Intra-ocular device for implanting in the capsular bag

(30) Priorité: 17.05.1991 FR 9106051
(43) Date de publication de la demande: 19.11.1992
(73) Titulaire: CORNEAL, F-75012 Paris (FR)
(72) Inventeur: Michel, Guy, F-44000 Nantes (FR)
(74) Mandataire: Dronne, Guy

(56) Documents cités:
- EP-A- 0 215 468
- US-A- 4 435 855
- US-A- 4 575 878
- US-A- 4 710 195
- US-A- 4 718 904

## Description

La présente invention a pour objet un dispositif intra-oculaire implantable dans le sac capsulaire de l'oeil.

Les dispositifs intra-oculaires pour implantation, plus couramment appelés implants intra-oculaires, connaissent un développement important. Ils constituent des systèmes correcteurs de la vision humaine qui peuvent, dans un certain nombre de cas, se substituer à des lentilles cornéennes ou à des verres correcteurs externes. Dans d'autres cas, ces implants peuvent être combinés par exemple avec des verres correcteurs pour obtenir une correction convenable de la vision. Un implant intra-oculaire se compose essentiellement d'une partie optique de forme générale circulaire ou légèrement ovalisée qui forme le système optique correcteur proprement dit. Un implant comporte également une partie haptique qui sert à la mise en place et à la fixation de la partie optique à l'intérieur de l'oeil.

Dans les dispositifs intra-oculaires les plus récents, cette partie haptique se compose le plus souvent de deux anses flexibles partant de la périphérie de la partie optique et dont les extrémités viennent au contact de la paroi interne de l'oeil pour assurer la mise en place de la partie optique et son centrage correct par rapport à l'axe oculaire de l'oeil humain. Les anses de la partie haptique doivent présenter une certaine élasticité pour pouvoir assurer une force de maintien suffisante pour assurer la mise en place du système optique. Le plus souvent également la partie optique et la partie haptique ne forment qu'une seule et même pièce usinée dans un matériau bio-compatible.

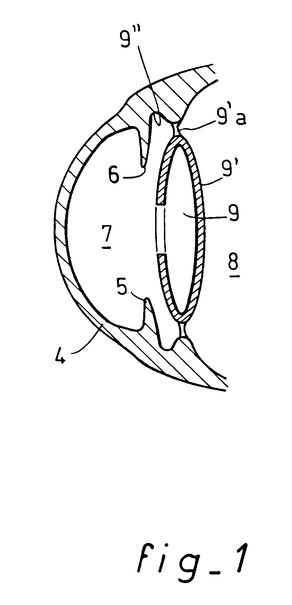

On peut distinguer trois types principaux d'implants intra-oculaires, selon la partie interne de l'oeil dans laquelle l'implant est mis en place. En se référant à la figure 1 qui est une vue en coupe verticale d'un oeil, on comprendra mieux les différents types d'implantation. Sur cette figure, on a représenté la cornée 4, ainsi que l'iris 5 qui définit ainsi la pupille 6. L'espace compris entre l'iris 5 et la partie avant de la cornée 4 limite la chambre antérieure 7. En arrière de l'iris 5, on trouve la chambre postérieure 8. Dans celle-ci, on trouve le cristallin 9 qui est enfermé dans le sac capsulaire 9′ dont la périphérie 9′a est solidaire de la cornée 4. Lorsqu'on est amené à pratiquer une opération de la cataracte, on procède à l'enlèvement du cristallin 9 qui peut se faire en laissant en place le sac capsulaire 9.

On comprend qu'ainsi la mise en place d'un implant peut se faire dans trois régions distinctes qui sont la chambre antérieure 7, la chambre postérieure 8 et le sac capsulaire 9′.

La présente invention concerne un implant intra-oculaire susceptible d'être mis en place dans le sac capsulaire 9′. Un des avantages de l'implantation dans le sac capsulaire 9′ consiste dans le fait que la partie aptique de l'implant est au contact du sac restant 9′ et non de la paroi interne de la cornée, ce qui évite ainsi les risques de lésion de la paroi interne de celle-ci. En outre, le diamètre interne du sac capsulaire est de l'ordre de 9 à 10 mm. En revanche, l'implantation dans le sac capsulaire est plus délicate, d'une part, parce que le diamètre interne du sac est relativement incertain, en général compris entre 9 et 10 mm, et que, d'autre part, le sac 9′ est relativement fragile. En conséquence, lors de la mise en place de l'implant dans le sac, il existe un risque que celui-ci ne soit déchiré et que la partie aptique de l'implant ne vienne au contact de la paroi interne de la cornée et plus précisément du sillon 9'', ce qui entraîne un mauvais centrage et une mauvaise fixation de l'implant, en raison de la différence de diamètre entre le sac capsulaire 9′ et le sillon 9''.

Une autre évolution importante actuelle de la chirurgie mise en oeuvre pour l'implantation intra-oculaire consiste dans le fait que l'on tend de plus en plus à pratiquer dans la cornée une incision de longueur aussi réduite que possible pour éviter l'apparition de phénomènes d'astigmatisme résultant de cette incision. Plus précisément, dans les techniques antérieures, on pratiquait une incision sur un angle d'environ 180°, ce qui permettait une introduction relativement aisée d'implants de diamètre et de dimension importantes mais entraînait trop fréquemment l'apparition d'astigmatisme. Selon les techniques plus récentes, on pratique une incision dans la cornée de l'ordre de 60°, ce qui correspond à une ouverture de 6 à 7 mm de largeur. On comprend qu'avec une telle technique il est nécessaire de disposer d'implants présentant des dimensions réduites par rapport à celles des implants utilisés précédemment.

On comprend que la technique d'implantation dans le sac capsulaire convient bien à ces techniques chirurgicales à incision réduite puisque le sac capsulaire a des dimensions réduites par rapport à la chambre antérieure ou à la chambre postérieure.

La mise en place de l'implant dans le sac capsulaire nécessite que les anses de la partie haptique présentent une élasticité importante pour éviter de déchirer le sac avec comme conséquence les inconvénients mentionnés précédemment. En outre, comme l'incision pratiquée pour l'introduction de l'implant à des dimensions réduites, il est nécessaire lors de cette introduction de replier de façon importante les anses pour que la dimension globale externe de l'implant soit inférieure aux dimensions de l'incision. Cette courbure des anses est obtenue en tenant l'implant et, plus précisément, ses anses à l'aide d'instruments chirurgicaux qui exercent localement sur ces anses une pression importante susceptible, sans précaution particulière, d'entraîner la rupture de l'extrémité des anses et donc de rendre inutilisable l'implant ainsi altéré.

Une autre difficulté consiste dans le fait que, avec des anses souples d'une longueur importante du type couramment utilisé, il est difficile de contrôler la déformation de ces anses après la mise en place de l'implant dans l'oeil. Il en résulte qu'il est difficile de contrôler le centrage correct de la partie optique du fait des possibilités de déformation dissymétrique des deux anses dans le cas où celles-ci ont à la fois une grande élasticité et une longueur relativement importante.

Les observations et essais déjà effectués ont montré que la transposition directe de la forme des anses couramment utilisées dans le cas d'implants pour chambre antérieure ou pour chambre postérieure ne permet pas d'obtenir des résultats satisfaisants avec des implants pour sac capsulaire. En effet, l'élasticité obtenue est le plus souvent insuffisante et le contact obtenu entre l'extrémité des anses et la périphérie du sac capsulaire n'est pas satisfaisant pour obtenir un centrage convenable de la partie optique dans le sac.

Le document US-A-4.435.855 décrit un implant intraoculaire notamment pour chambre antérieure dont les caractéristiques sont reprises dans le préambule de la revendication 1.

Un objet de l'invention est de fournir un dispositif intra-oculaire implantable dans le sac capsulaire qui permet, d'une part, une implantation à l'aide d'une incision dans la cornée de longueur réduite et qui, d'autre part, permet un maintien et un centrage de la partie optique corrects dans le sac capsulaire.

Pour atteindre ce but, le dispositif intra-oculaire pour implantation dans le sac capsulaire, selon l'invention, comprend une partie optique de forme sensiblement circulaire et une partie haptique consistant en deux anses flexibles incurvées formant une seule pièce avec ladite partie optique, chacune présentant une première extrémité libre destinée à venir en contact avec la paroi du sac capsulaire et une deuxième extrémité raccordée à la périphérie de la partie optique par des congés, lesdites deuxièmes extrémités étant sensiblement diamétralement opposées, et il se caractérise en ce que la partie optique a un diamètre compris entre 4,5 et 6 mm, en ce que l'ensemble des deux anses, au repos, est situé à l'intérieur d'un cercle concentrique à la partie optique et présentant un diamètre compris entre 11,5 et 13 mm et en ce que chaque anse présente une courbure sensiblement régulière, la fibre moyenne faisant, dans la zone de raccordement à la périphérie de la partie optique, un angle b avec la tangente à celle-ci compris entre 25 et 35°, chaque anse présentant une partie courante de largeur e sensiblement constante et une partie terminale dont la largeur e est comprise entre 1,4e et 1,8e.

Avec la caractéristique citée en dernier lieu dans la revendication 1, on obtient des anses dont l'extrémité est renforcée permettant ainsi l'utilisation des instruments chirurgicaux avec une grande sécurité et sans risque de rupture tout en autorisant une courbure et une flexibilité suffisantes en localisant la courbure dans la partie courante de l'anse.

D'autres caractéristiques et avantages de l'invention apparaîtront plus clairement à la lecture de la description qui suit d'un mode préféré de réalisation de l'invention, donné à titre d'exemple non limitatif. La description se réfère aux figures annexées sur lesquelles :
- la figure 1, déjà décrite, montre en coupe longitudinale un oeil humain afin de montrer les différentes possibilités d'implantation ;
- la figure 2 est une vue de face d'un dispositif intra-oculaire selon l'invention ; et
- la figure 3 est une vue de côté de l'implant de la figure 2.

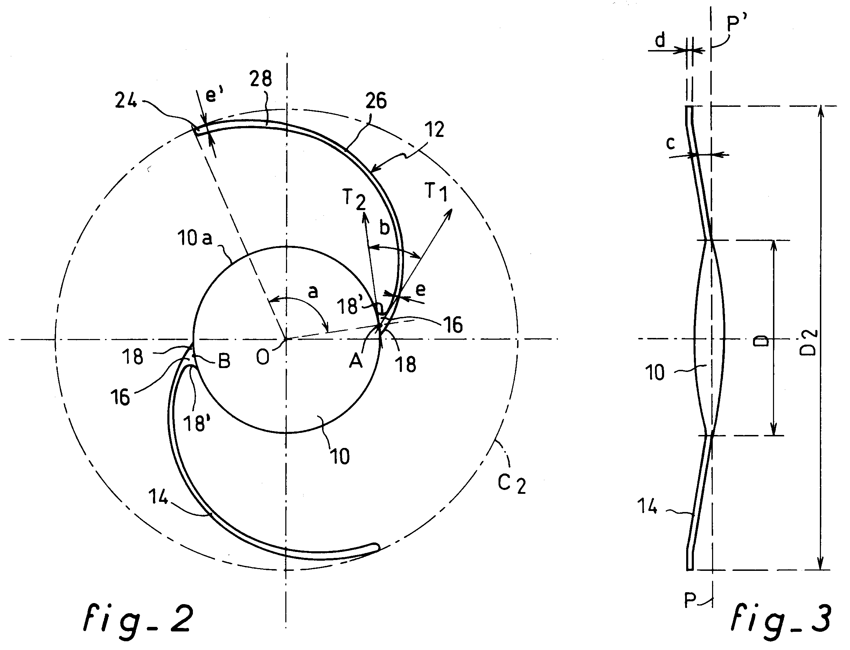

En se référant aux figures 2 et 3, on va décrire un mode préféré de réalisation du dispositif intra-oculaire pour implantation dans le sac capsulaire, que l'on appellera ultérieurement par simplification implant. L'implant comprend une partie optique constituée par une lentille convergente 10 de centre 0 et de diamètre D. Dans l'exemple particulier considéré, le diamètre est égal à 5 mm mais de façon plus générale ce diamètre peut être compris entre 4,5 et 6 mm. La partie haptique de l'implant est constituée par deux anses respectivement référencées 12 et 14, chaque anse part de la périphérie 10a de la lentille 10 en des points A et B qui sont diamétralement opposés. Les deux anses 12 et 14 sont sensiblement identiques et on se contentera donc de décrire en détail l'anse 12. L'anse 12 comporte une première extrémité 16 de raccordement à la périphérie 10a de la lentille 10. L'anse est raccordée par deux congés 18, 18′ de telle manière que cette extrémité a une largeur très sensiblement supérieure à celle e de la partie courante 26 de l'anse. La figure 2 montre que l'anse 12 a une courbure régulière. Celà ne signifie pas que le rayon de courbure est le même pour toute l'anse mais que d'un point à un autre les variations du rayon de courbure sont régulières et faibles.

La figure 2 montre également que dans la zone de raccordement (point A) de l'anse 12 à la périphérie de la lentille 10, la tangente T1 à la fibre neutre de l'anse fait un angle b avec la tangente T2 à la périphérie de la lentille 10 en ce même point. Dans le mode de réalisation considéré, l'angle b vaut 30°. Plus généralement l'angle b est compris entre 25 et 35°. On voit également que la largeur e de la portion courante 26 de l'anse, c'est-à-dire sa dimension dans le plan de la lentille 10 est sensiblement inférieure à la largeur e′ de la portion terminale d'extrémité 28. Conformément à la revendication 1, e' est entre 1,4 fois e et 1,8 fois e. Dans l'exemple considéré, e est égal à 0,11 mm et e′ est égal à 0,18 mm.

En outre, on voit que l'ensemble des anses 12 et 14 est inscrit à l'intérieur d'un cercle C2 concentrique à la lentille 10 dont le diamètre D2 est égal, dans l'exemple considéré, à 12 mm. Plus généralement, le diamètre D2 est compris entre 11,5 et 13 mm. Bien sûr, sur la figure 2, on a représenté les anses 12 et 14 au repos, c'est-à-dire dans leur position où elles ne sont soumises à aucun effort de flexion. On note également que l'extrémité terminale 24 de chacune des anses se termine avec une légère angulation par rapport au cercle C2. Plus précisément, cet angle est déterminé pour que, lorsque l'implant est mis en place dans le sac 9′, la portion terminale de chaque anse fléchie soit sensiblement tangente à la périphérie de la paroi interne du sac, c'est-à-dire à un cercle de diamètre compris entre 9 et 10 mm.

La figure 2 montre également l'angle au centre a sous lequel l'anse 12 est vue. Dans le cas particulier considéré, l'angle a est égal à 105°. Plus généralement, l'angle a est compris entre 95 et 110°.

Si l'on se réfère maintenant à la figure 3, on voit que l'épaisseur d de chaque anse 12 ou 14 est sensiblement constante sur toute la longueur des anses et vaut dans le cas particulier 0,15 mm. En outre, on voit que la direction générale de chaque anse fait un angle c avec le plan PP' de la lentille 10. Cet angle c est dans l'exemple considéré égal à 10 degrés.

Il découle de la description précédente que l'implant selon l'invention s'adapte parfaitement à l'implantation dans le sac capsulaire selon la technique de l'incision réduite. Du fait que, à son départ, chaque anse fasse un angle b compris entre 25 et 35°, elle présente un angle qui n'est ni trop important, ce qui limiterait les possibilités de flexion dans sa partie courante 26, en augmentent la raideur selon la direction radiale de la lentille, ni trop faible, ce qui limiterait l'amplitude du débattement de l'extrémité 24 de l'anse et donc les possibilités d'adaptation à la plage de diamètres que peuvent présenter les sacs capsulaires. En outre, du fait que les parties terminales des anses ont une largeur e' sensiblement supérieure à celle de la portion courante, on obtient dans les portions terminales une résistance mécanique supérieure tout en autorisant grâce à la largeur réduite de la portion courante des anses une flexion satisfaisante des anses localisée dans cette partie des anses. Du fait de cette portion de largeur accrue, il est possible de saisir efficacement l'implant à l'aide d'instruments chirurgicaux sans risquer d'entraîner la rupture des anses. De plus cette portion de largeur plus importante diminue sensiblement les risques de percer le sac capsulaire.

En outre, la longueur totale de chaque anse est relativement réduite par rapport aux implants de type connu à anse souple grâce à la valeur relativement limitée de l'angle au centre a. Cette longueur relativement réduite de chaque anse permet de mieux contrôler la déformation élastique de ces anses pour se conformer au diamètre interne du sac capsulaire tout en maintenant un bon centrage de l'implant.

## Revendications

1. Dispositif intraoculaire pour implantation dans le sac capsulaire comprenant une partie optique (10) de forme sensiblement circulaire et une partie haptique consistant en deux anses flexibles incurvées (12, 14) formant une seule pièce avec ladite partie optique, chacune présentant une première extrémité libre destinée à venir en contact avec la paroi du sac capsulaire et une deuxième extrémité raccordée à la périphérie de la partie optique par des congés (18, 18') lesdites deuxièmes extrémités étant sensiblement diamétralement opposées, caractérisé en ce que la partie optique (10) a un diamètre compris entre 4,5 et 6 mm, en ce que l'ensemble des deux anses (12, 14), au repos, est situé à l'intérieur d'un cercle (C2) concentrique à la partie optique et présentant un diamètre (D2) compris entre 11,5 et 13 mm et en ce que chaque anse (12, 14) présente une courbure sensiblement régulière, la fibre moyenne faisant, dans la zone de raccordement à la périphérie de la partie optique, un angle b avec la tangente à celle-ci compris entre 25 et 35°, chaque anse (12, 14) présentant une partie courante de largeur e sensiblement constante et une partie terminale (28) dont la largeur e est comprise entre 1,4e et 1,8e.

2. Dispositif intraoculaire selon la revendications 1, caractérisé en ce que ladite extrémité terminale (28) fait avec ledit cercle (C2) un angle tel que, lorsque ledit dispositif est mis en place dans le sac capsulaire, ladite portion terminale fléchie soit sensiblement tangente à un cercle de diamètre compris entre 9 et 10 mm correspondant aux dimensions internes du sac capsulaire.

3. Dispositif intraoculaire selon l'une quelconque des revendications 1 et 2, caractérisé en ce que lesdites anses (12, 14) ont une épaisseur sensiblement constante.

4. Dispositif intraoculaire selon l'une quelconque des revendications 1 à 3, caractérisé en ce que l'angle au centre (a) sous lequel est vue chaque anse est compris entre 95 et 110°.

5. Dispositif intraoculaire selon l'une quelconque des revendications 1 à 4, caractérisé en ce que ladite première extrémité de chaque anse (12, 14) est raccordée à la périphérie de la partie optique (10) par des congés (18, 18') donnant à ladite première extrémité de l'anse une largeur très sensiblement supérieure à celle de la partie courante (26) de l'anse.

6. Dispositif intraoculaire selon l'une quelconque des revendications 1 à 5, caractérisé en ce que chaque anse (12, 14) fait avec le plan (PP') de la partie optique (10) un angle de l'ordre de 10 degrés.

## Claims

1. An intraocular device for implantation in the capsular sac, comprising an optical part (10) substantially circular in shape and a haptic part consisting of two flexible curved arms (12, 14) forming a single piece with said optical part, each having a first free extremity intended to come into contact with the wall of the capsular sac and a second extremity connected to the periphery of the optical part by fillets (18, 18'), said second extremities being substantially diametrically opposite, characterised in that the optical part (10) has a diameter of between 4.5 and 6 mm, in that the two arms (12, 14) together, when at rest, are located within a circle (C2) concentric to the optical part and having a diameter (D2) of between 11.5 and 13 mm and in that each arm (12, 14) has a substantially regular curvature, the axis forming, in the area of connection to the periphery of the optical part, an angle b with the tangent to said optical part of between 25 and 35°, each arm (12, 14) having an extended part (26) of a substantially constant width e, and a terminal part (28), the width e' of which is between 1.4e and 1.8e.

2. An intraocular device according to Claim 1, characterized in that said terminal extremity (28) forms an angle with said circle (C2) such that, when said device is positioned in the capsular sac, said terminal portion, being flexed, is substantially tangential to a circle with a diameter of between 9 and 10 mm corresponding to the internal dimensions of the capsular sac.

3. An intraocular device according to any one of Claims 1 and 2, characterised in that said arms (12, 14) have a substantially constant thickness.

4. An intraocular device according to any one of Claims 1 to 3, characterised in that the angle at the centre (a) over which each arm is seen is comprised between 95 and 110°.

5. An intraocular device according to any one of Claims 1 to 4, characterised in that said first extremity of each arm (12, 14) is connected to the periphery of the optical part (10) by fillets (18, 18') giving said first extremity of the arm a width very substantially greater than that of the extended part (26) of the arm.

6. An intraocular device according to any one of Claims 1 to 5, characterised in that each arm (12, 14) forms an angle with the plane (PP') of the optical part (10), of the order of 10 degrees.

## Patentansprüche

1. Intraokulare Vorrichtung für die Implantation in dem Kapselsack, enthaltend:
einen optischen Teil (10) mit im wesentlichen kreisförmiger Form, und
einen Halteteil mit zwei gebogenen flexiblen Bügeln (12, 14), die als ein einziges Teil mit dem optischen Teil ausgebildet sind, von denen jede ein erstes freies Ende aufweist, das in Kontakt mit der Wand des Kapselsacks gelangt, und ein zweites Ende, das mit einem Rand des optischen Teils über Ausrundungen (18, 18') verbunden ist, wobei die zweiten Enden im wesentlichen diametral einander gegenüberliegen,
dadurch **gekennzeichnet,** daß
der optische Teil (10) einen Durchmesser zwischen 4,5 und 6 mm aufweist,
die beiden Bügel (12, 14) in ihrer Ruhestellung im Inneren eines Kreises (C2) angeordnet sind, der konzentrisch zu dem optischen Teil verläuft und einen Durchmesser (D2) zwischen 11,5 und 13 mm aufweist, und
jeder Bügel (12, 14) eine im wesentlichen gleichmäßige Krümmung aufweist,
die mittlere Faser am Verbindungsbereich des Randabschnitts des optischen Teils einen Winkel von b mit der Tangente mit diesem einschließt, der zwischen 25 und 35° liegt,
jeder Bügel (12, 14) einen gebogenen Abschnitt mit einer Breite e aufweist, die im wesentlichen konstant ist, und einen Endabschnitt (28), dessen Breite e' zwischen 1,4 x e und 1,8 x e liegt.

2. Intraokulare Vorrichtung nach Anspruch 1, dadurch gekennzeichnet, daß der Endabschnitt (28) mit dem Kreis (C2) einen Winkel derart einschließt, daß bei Abordnung der Vorrichtung im Kapselsack der gebogene Endabschnitt im wesentlichen tangential an einem Kreis mit einem Durchmesser zwischen 9 und 10 mm gemäß den Innenabmessungen des Kapselsacks anliegt.

3. Intraokulare Vorrichtung nach Anspruch 1 oder 2, dadurch gekennzeichnet, daß die Bügel (12, 14) eine im wesentlichen konstante Dicke auweisen.

4. Intraokulare Vorrichtung nach einem der Ansprüche 1 bis 3, dadurch gekennzeichnet, daß der Mittenwinkel (a), unter dem jeder Bügel gesehen wird, in dem Bereich zwischen 95°und 110° liegt.

5. Intraokulare Vorrichtung nach einem der Ansprüche 1 bis 4, dadurch gekennzeichnet, daß das erste Ende jedes Bügels (12, 14) mit einem Rand des optischen Teils (10) über Ausrundungen (18, 18') verbunden ist, die dem ersten Ende des Bügels eine Breite verleihen, die erheblich größer als diejenige des gebogenen Abschnitts (26) des Bügels ist.

6. Intraokulare Vorrichtung nach einem der Ansprüche 1 bis 5, dadurch gekennzeichnet, daß jeder der Bügel (12, 14) mit der Ebene (PP') des optischen Teils (10) einen Winkel in der Größenordnung von 10° einschließt.
